# EUROPEAN PATENT APPLICATION

(11) **EP 4 023 183 A1**
(43) Date of publication of application: **06.07.2022**
(21) Application number: 20217534.5
(22) Date of filing: 29.12.2020
(51) Int. Cl.: A61B 34/30, A61B 34/00, B25J 9/12, A61M 25/01

(54) **CATHETER ROBOT COMPRISING CATHETER TRANSLATION MODULES FOR FLEXIBLE ELONGATED MEDICAL ELEMENTS**

(71) Applicant: Robocath, 76000 Rouen (FR)
(72) Inventor: FOURNIER, Bruno, 41100 Saint Ouen (FR); BOULANGE, Marc, 76740 La Gaillarde (FR); BELOUIN, Alexis, 44522 Pouillé Les Coteaux (FR)
(74) Representative: Plasseraud IP

(57) **Abstract**

This invention relates to a catheter robot comprising: a base (100), at least 2 catheter translation modules (1, 2) which are supported by said base (100), each catheter translation module (1, 2) comprising: only one set (7, 8) of movable parts (71 to 74, 81 to 84) so as to translate longitudinally at a time only one flexible elongated medical element, a group of several crossing tracks (3 to 6) within which several corresponding flexible elongated medical elements can translate through said catheter translation module (1, 2), a switch (61, 62) to direct said single set (7, 8) of movable parts (71 to 74, 81 to 84) in any of said crossing tracks (3 to 6) so as to then translate longitudinally said corresponding flexible elongated medical element, said first and second catheter translation modules (1, 2) being longitudinally spaced apart from each other so that: respective groups of crossing tracks (3 to 6) of both catheter translation modules (1, 2) are facing each other so that a given flexible elongated medical element which translates within a crossing track (3 to 6) of one group also translates within a crossing track (3 to 6) of the other group, both switches (61, 62) of said first and second catheter translation modules (1, 2) are synchronized together so as not to direct their respective sets (7, 8) of movable parts (71 to 74, 81 to 84) in crossing tracks (3 to 6) of said first and second groups which face each other.

## Description

### FIELD OF THE INVENTION

The invention relates to the technical field of catheter robots comprising catheter translation modules structured and disposed so as to translate one or more flexible elongated medical elements.

### BACKGROUND OF THE INVENTION

According to a prior art, a catheter robot comprises several flexible elongated medical elements, for instance, either catheter or guidewire. This catheter robot comprises an internal mechanism, made of movable parts, to translate these flexible elongated medical elements. However, exchange from one flexible elongated medical element to the other, has to be performed manually by the practitioner. This manual exchange requires an additional step to be performed by a qualified practitioner, thereby losing time and energy.

Indeed, a simple Percutaneous Coronary Intervention usually requires one guidewire and one catheter or even one balloon catheter. This catheter balloon could be a bare balloon or a balloon with a stent, but here the term balloon catheter will be used to name both. These guide wire and balloon catheter are to be inserted in the patient at the same time. In more complex cases, it may even become useful to have more flexible elongated medical elements inserted simultaneously. For example, in the case of a bifurcation, it may become interesting to use two guidewires and two balloon catheters.

Such a catheter robot can manipulate two flexible elongated medical elements robotically at the same time. In order to use more flexible elongated medical elements, such a catheter robot has a "parking lot" enabling to keep the flexible elongated medical elements inserted in the patient as well as maintaining them immobile, by using a clamp, while enabling two more flexible elongated medical elements, for instance a guidewire and a balloon catheter, to be inserted in the robotized tracks at the same time. If the user wants to manipulate a flexible elongated medical element in the parking lot, he needs to exchange this flexible elongated medical element with the corresponding flexible elongated medical element in the robotized track: a guidewire has to be exchanged with a guidewire and a balloon catheter with a balloon catheter, since the guidewire track and the balloon track are different and dedicated.

This manipulation is problematic, because it must be done manually on the catheter robot. Indeed, this manual manipulation requires a dedicated person and adds an extra step to the procedure. Such a dedicated manual step presents the inconvenient to increase the whole procedure time. In addition, in case the catheter robot design implies a curvature of the balloon, this could limit the push force and reduce the balloon catheter maneuverability.

### SUMMARY OF THE INVENTION

The object of the present invention is to alleviate at least partly the above-mentioned drawbacks.

More particularly, the invention aims at proposing a more automated catheter robot, allowing for performing such exchange between two different flexible elongated medical elements automatically. Therefore, according to the invention, it is useful and interesting to provide for catheter translation modules which comprise each an internal translation mechanism made of one set of movable parts structured to translate one flexible elongated medical element, and which comprise each more than one track to receive such flexible elongated medical element.

Indeed, the track containing the flexible elongated medical element being translated at the moment by this set of movable parts is a robotized track while other tracks are parking tracks. Besides, this set of movable parts can automatically change from one track to the other, to start translating another flexible elongated medical element, thereby changing at that instant the robotized track into a parking track and one of the parking tracks into the new robotized track.

It is proposed then several tracks able to receive several corresponding flexible elongated medical elements, with an internal translation mechanism made of one set of movable parts, this set of movable parts being able to move from one track to the other, to translate one or the other among several flexible elongated medical elements, giving to each track where it goes the status of the new robotized track while all other tracks keep or get the status of parking track where no translation of flexible elongated medical element can be performed at that moment.

This object is achieved with a catheter robot comprising: a base, at least 2 catheter translation modules which are supported by said base, each catheter translation module comprising: only one set of movable parts so as to translate longitudinally at a time only one flexible elongated medical element, a group of several crossing tracks within which several corresponding flexible elongated medical elements can translate through said catheter translation module, a switch to direct said single set of movable parts in any of said crossing tracks so as to then translate longitudinally said corresponding flexible elongated medical element, said first and second catheter translation modules being longitudinally spaced apart from each other so that: respective groups of crossing tracks of both catheter translation modules are facing each other so that a given flexible elongated medical element which translates within a crossing track of one group also translates within a crossing track of the other group, both switches of said first and second catheter translation modules are synchronized together so as not to direct their respective sets of movable parts in crossing tracks of said first and second groups which face each other.

This object is more precisely achieved with a catheter robot comprising: a base, at least 2 catheter translation modules supported by said base, each catheter translation module comprising: only one set of two pairs of movable pads: said pads of a same pair at least partly facing each other, alternatively, each of said two pairs clamping between its pads a flexible elongated medical element so as to then translate longitudinally said flexible elongated medical element, a group of several crossing tracks within which several corresponding flexible elongated medical elements can translate through said catheter translation module, a switch to direct said single set of two pairs of movable pads in any of said crossing tracks so as to then translate longitudinally said corresponding flexible elongated medical element, said first and second catheter translation modules being longitudinally spaced apart from each other so that: respective groups of crossing tracks of both catheter translation modules are facing each other so that a given flexible elongated medical element which translates within a crossing track of one group also translates within a crossing track of the other group, both switches of said first and second catheter translation modules are synchronized together so as not to direct their respective sets of two pairs of movable pads in crossing tracks of said first and second groups which face each other.

Preferably, the catheter robot according to the invention also comprises: a clamping device which is longitudinally spaced apart from said first and second catheter translation modules and which comprises: a group of several crossing tracks within which several corresponding flexible elongated medical elements can translate through said catheter translation modules, said group of crossing tracks facing at least one of said groups of crossing tracks of catheter translation modules so that a given flexible elongated medical element which translates within a crossing track of both groups of said catheter translation modules also translates within a crossing track of said group of said clamping device, said clamping device being synchronized with said catheter translation modules: so as not to clamp in any crossing track facing a crossing track of said catheter translation module within which any of said sets of two pairs of movable pads is directed by any of said switches, so as to clamp in one or more or all crossing tracks not facing a crossing track of said catheter translation module within which any of said sets of two pairs of movable pads is directed by any of said switches.

So, at a given time, there are several tracks able to receive several corresponding flexible elongated medical elements, with an internal translation mechanism made of one set of movable parts, this set of movable parts being able to move from one track to the other, to translate one or the other among several flexible elongated medical elements.

But at this given time, there is only one robotized track where this set of movable parts translates one among the several flexible elongated medical elements. All other tracks are, at this given time, only parking tracks where no translation of flexible elongated medical element can be performed at that moment. In these parking tracks, the flexible elongated medical elements are unclamped since they are not clamped by this set of movable parts.

However, it may be dangerous, for the patient within which arteries or veins the flexible elongated medical elements are to be inserted, to leave one or more of these flexible elongated medical elements completely free to move. Therefore, this additional clamping device is provided for clamping one or more or preferably all the flexible elongated medical elements which are unclamped, i.e. which are not clamped by the set of movable parts of the internal translation mechanism of a catheter translation module. Thereby, this additional clamping device provides for additional security, in a simple and efficient way.

More preferably, the catheter robot according to the invention also comprises: a clamping device which is longitudinally spaced apart from said first and second catheter translation modules and which comprises: a group of several crossing tracks within which several corresponding flexible elongated medical elements can translate through said catheter translation module, said group of crossing tracks facing at least one of said groups of crossing tracks of catheter translation modules so that a given flexible elongated medical element which translates within a crossing track of both groups of said catheter translation modules also translates within a crossing track of said group of said clamping device, said clamping device being synchronized with said catheter translation modules: so as not to clamp in any crossing track facing a crossing track of said catheter translation module within which any of said sets of two pairs of movable pads is directed by any of said switches, so as to clamp in one or more crossing tracks not facing a crossing track of said catheter translation module within which any of said sets of two pairs of movable pads is directed by any of said switches.

Preferred embodiments comprise one or more of the following features, which can be taken separately or together, either in partial combination or in full combination.

Preferably, said clamping device comprises a cam mechanism disposed so as: to require motor energy to change the status of anyone of said crossing tracks from clamped to unclamped or from unclamped to clamped, not to require motor energy to maintain the status of anyone of said crossing tracks, either clamped or unclamped.

Hence, the clamping device structure presents a better compromise between efficiency and simplicity as well as working cost, since motor energy is only needed to exchange status, between clamped and unclamped, and not to maintain status, either clamped or unclamped.

Preferably, said cam mechanism comprises a single cam with different diameters disposed along its rotation axis, so as: to clamp in only two crossing tracks at any rotation angle position, to clamp in different combinations of two crossing tracks respectively for different rotation angle ranges, each possible combination of two crossing tracks corresponding respectively to an associated rotation angle range.

Hence, the clamping device structure presents an even better compromise between efficiency and simplicity as well as working cost, since a single rotating motor is needed to perform all status exchanges, between clamped and unclamped, and to maintain all status, either clamped or unclamped.

Preferably, each catheter translation module is also a catheter rotation module so as to rotate around longitudinal axis said flexible elongated medical element.

Hence, the flexible elongated medical elements, not only can be translated, but also can be rotated, and besides advantageously by the same set of movable parts which, not only is able to translate the flexible elongated medical element, but also is able to rotate this flexible elongated medical element, what improves the compromise between simplicity and efficiency of the catheter robot.

Preferably, said clamping device comprises a cam mechanism disposed so as: to require motor energy to change the status of anyone of said crossing tracks from clamped to unclamped or from unclamped to clamped, not to require motor energy to maintain the status of anyone of said crossing tracks, either clamped or unclamped.

Hence, the clamping device structure presents a better compromise between efficiency and simplicity as well as working cost, since motor energy is only needed to exchange status, between clamped and unclamped, and not to maintain status, either clamped or unclamped.

Preferably, said cam mechanism comprises a single cam with different diameters disposed along its rotation axis, so as: to clamp in only two crossing tracks at any rotation angle position, to clamp in different combinations of two crossing tracks respectively for different rotation angle ranges, each possible combination of two crossing tracks corresponding respectively to an associated rotation angle range.

Hence, the clamping device structure presents an even better compromise between efficiency and simplicity as well as working cost, since a single rotating motor is needed to perform all status exchanges, between clamped and unclamped, and to maintain all status, either clamped or unclamped.

Preferably, each catheter translation module is also a catheter rotation module: alternatively, each of said two pairs clamping between its facing pads a flexible elongated medical element so as to then rotate around longitudinal axis said flexible elongated medical element, by making said flexible elongated medical element rolling between its facing pads.

Hence, the flexible elongated medical elements, not only can be translated, but also can be rotated, and besides advantageously by the same set of movable parts which, not only is able to translate the flexible elongated medical element, but also is able to rotate this flexible elongated medical element, what improves the compromise between simplicity and efficiency of the catheter robot.

Preferably, said group of several crossing tracks comprises at least 4 crossing tracks, and preferably comprises only 4 crossing tracks.

Hence, a single catheter translation module can manage alternatively many flexible elongated medical elements.

Preferably, said crossing tracks receive respectively: a first catheter, preferably a catheter balloon or a catheter stent, a first guide of said first catheter, a second catheter, preferably a catheter balloon or a catheter stent, a second guide of said second catheter.

Hence, a single catheter translation module can manage alternatively two complete sets of flexible elongated medical elements, each set of flexible elongated medical elements comprising a catheter and its guidewire guiding said catheter.

Preferably, said first and second catheter translation modules are structurally identical to each other.

Hence, the catheter robot is globally made simpler while it still remains as efficient as requested.

Preferably, each track of one or more or all of the catheter translation modules is flexible elongated medical element agnostic so that said track can handle either a catheter or a guidewire.

Hence, the catheter robot is globally made simpler while it still remains as efficient as requested.

Further features and advantages of the invention will appear from the following description of embodiments of the invention, given as non-limiting examples, with reference to the accompanying drawings listed hereunder.

### BRIEF DESCRIPTION OF THE DRAWINGS

Fig. 1 shows schematically an example of part of a catheter robot according to an embodiment of the invention.
Fig. 2a-2b-2c-2d show schematically respectively four different states of the internal translation mechanism, in a plane, of an example of a catheter robot according to an embodiment of the invention.
Fig. 3a-3b-3c-3d show schematically respectively four different states of the internal translation mechanism, in another plane, of an example of a catheter robot according to an embodiment of the invention.
Fig. 4 shows schematically an example of a catheter robot according to an embodiment of the invention.
Fig. 5 shows schematically an example of a clamping device of a catheter robot according to an embodiment of the invention.
Fig. 6 shows schematically a first example of an internal clamping mechanism of a clamping device of a catheter robot according to an embodiment of the invention.
Fig. 7 shows schematically a second example of an internal clamping mechanism of a clamping device of a catheter robot according to an embodiment of the invention.
Fig. 8 shows schematically a third example of an internal clamping mechanism of a clamping device of a catheter robot according to an embodiment of the invention.

### DETAILED DESCRIPTION OF THE INVENTION

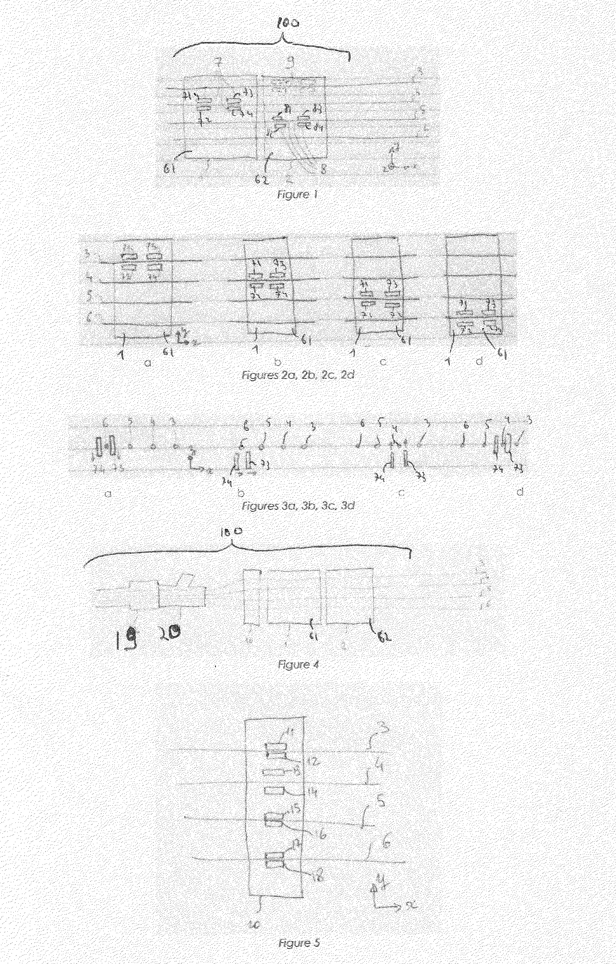

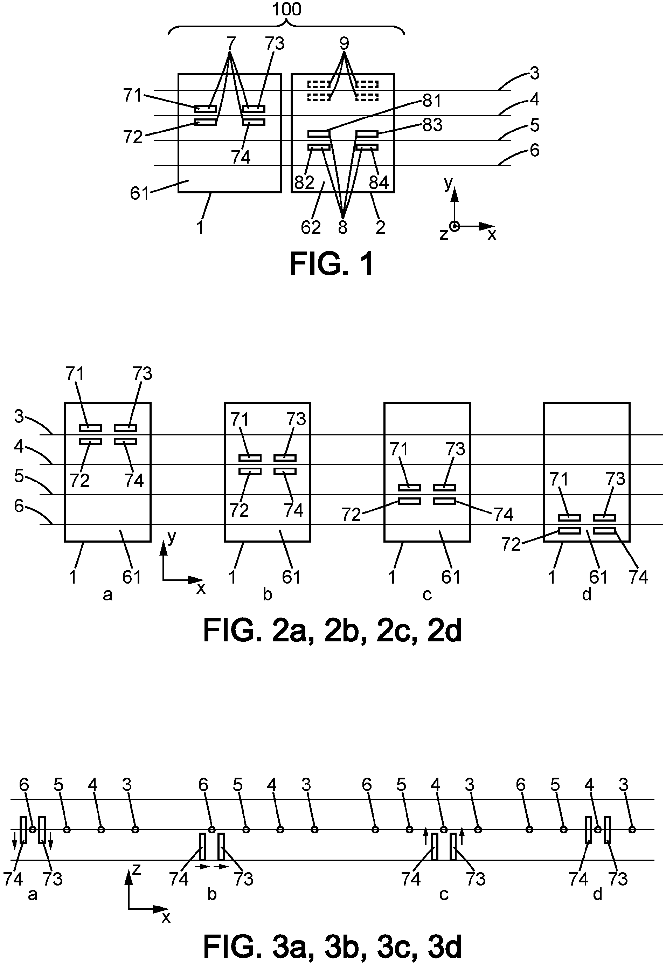

Fig. 1 shows schematically an example of part of a catheter robot according to an embodiment of the invention.

The catheter robot comprises a base 100, at least 2 catheter translation modules 1 and 2 supported by this base 100. The first 1 and second 2 catheter translation modules are structurally identical to each other.

The group of several crossing tracks 3, 4, 5 and 6, comprises at least 4 crossing tracks 3, 4, 5 and 6, and preferably comprises only 4 crossing tracks 3, 4, 5 and 6. These crossing tracks receive respectively, for example, a first catheter, preferably a balloon catheter or a catheter stent, in crossing track 4, a first guide of said first catheter, in crossing track 6, a second catheter, preferably a balloon catheter or a catheter stent, in crossing track 3, a second guide of said second catheter, in crossing track 5. Each track 3 to 6 is device agnostic, i.e. it can handle either a guidewire or a balloon catheter as well.

The catheter translation module 1 comprises only one set 7 of two pairs of movable pads 71, 72, 73 and 74, the pads 71 and 72, as well as 73 and 74, of a same pair at least partly facing each other. Alternatively, each of said two pairs clamps between its pads, either 71 and 72, or 73 and 74, a flexible elongated medical element, here in the crossing track 4, so as to then translate longitudinally this flexible elongated medical element. The catheter module 1 also comprises a group of several crossing tracks 3, 4, 5 and 6, within which several corresponding flexible elongated medical elements can translate through this catheter translation module 1. The catheter module 1 still comprises a switch 61 to direct this single set 7 of two pairs of movable pads 71 to 74 in any of said crossing tracks 3 to 6, so as to then translate longitudinally this corresponding flexible elongated medical element, here in crossing track 4.

The catheter translation module 1 is also a catheter rotation module. Indeed, alternatively, each of said two pairs clamping between its facing pads, 71 and 72 then 73 and 74, a flexible elongated medical element so as to then rotate around longitudinal axis this flexible elongated medical element, by making this flexible elongated medical element rolling between its facing pads, either 71 and 72, or 73 and 74, like a tube or a cigarette rolling between the fingers of a hand.

The catheter translation module 2 comprises only one set 8 of two pairs of movable pads 81, 82, 83 and 84, the pads 81 and 82, as well as 83 and 84, of a same pair at least partly facing each other. Alternatively, each of said two pairs clamps between its pads, either 81 and 82, or 83 and 84, a flexible elongated medical element, here in the crossing track 5, or in the crossing track 3 in its position 9, so as to then translate longitudinally this flexible elongated medical element. The catheter module 2 also comprises a group of several crossing tracks 3, 4, 5 and 6, within which several corresponding flexible elongated medical elements can translate through this catheter translation module 2. The catheter module 2 still comprises a switch 62 to direct this single set 8 of two pairs of movable pads 81 to 84 in any of said crossing tracks 3 to 6, so as to then translate longitudinally this corresponding flexible elongated medical element, here in crossing track 5, or in crossing track 3 when in its position 9.

The catheter translation module 2 is also a catheter rotation module. Indeed, alternatively, each of said two pairs clamping between its facing pads, 81 and 82 then 83 and 84, a flexible elongated medical element so as to then rotate around longitudinal axis this flexible elongated medical element, by making this flexible elongated medical element rolling between its facing pads, either 81 and 82, or 83 and 84, like a tube or a cigarette rolling between the fingers of a hand.

The first 1 and second 2 catheter translation modules are longitudinally spaced apart from each other, so that respective groups of crossing tracks 3 to 6 of both catheter translation modules 1 and 2 are facing each other, so that a given flexible elongated medical element which translates within a crossing track, either 3 or 4 or 5 or 6, of one group also translates within the same crossing track, either 3 or 4 or 5 or 6, of the other group.

Both switches 61 and 62 of first 1 and second 2 catheter translation modules are synchronized together so as not to direct their respective sets 7 and 8 of two pairs of movable pads, respectively 71 to 74 and 81 to 84, in crossing tracks 3 to 6 of these first and second groups which face each other. For instance, in first catheter translation module 1, a flexible elongated medical element is located in crossing track 4, whereas in second catheter translation module 2, flexible elongated medical elements are respectively located in crossing tracks 3 and 5.

The catheter translation modules 1 and 2 are robotic modules, to be used in a catheter robot dedicated rather to vascular interventions with rapid exchange flexible elongated medical elements, in which up to 4 flexible elongated medical elements can be inserted, thank to 4 linear, i.e. non-curved, parallel tracks 3 to 6, where any of the 4 tracks 3 to 6 can be selected as the robotized track, for instance track 4 in catheter translation module 1 on figure 1, the 3 other tracks being in "parking" mode, for instance tracks 3, 5 and 6 in catheter translation module 1 on figure 1, i.e. where the flexible elongated medical elements remain immobile. On catheter translation module 2, either the robotized track is track 5, with set 8 of pairs of movable pads 81 to 84, other tracks 3, 4 and 6 being parking tracks, or the robotized track becomes track 3, with set 8 of pairs of movable pads in its position 9, other tracks 4, 5 and 6 then becoming parking tracks. The change to position 9 is performed by switch 62.

The 2 modules 1 and 2 can be combined in order to have a 4-track system in which 2 tracks are robotized, tracks 4 and 5 on figure 1, and 2 tracks in parking mode, tracks 3 and 6 on figure 1.

The selection of the robotized tracks, among tracks 3 to 6, is done by the user or by the practitioner, thanks to any Man Machine Interface device, for instance button, touchscreen, joystick, etc..., and is automatically executed by the switches 61 and 62 of the catheter translation modules 1 and 2 of the catheter robot in a few seconds or even less.

This two-module configuration is shown on figure 1. The system is composed of two identical catheter translation modules 1 and 2. Each catheter translation module 1 or 2 comprises an electro-mechanical system, not represented here for sake of simplicity, allowing each set 7 or 8 of 4 pads, 71 to 74 for set 7, or 81 to 84 for set 8, to be translated along the x, y and z axis while maintaining their orientation constant.

Appropriate movements of the pads 71 to 74 and 81 to 84 can create a translation, a rotation, or a combined rotation of the flexible elongated medical element that they respectively clamp. This has already been described in patent applications FR3022147 and FR3037269, hereby incorporated by reference.

Fig. 2a-2b-2c-2d show schematically respectively four different states of the internal translation mechanism, in a plane, of an example of a catheter robot according to an embodiment of the invention.

As compared to the pad motion described in the aforementioned documents, patent applications FR3022147 and FR3037269, a novelty, as shown here on figures 2a to 2d, lies in the possibility to have a longer stroke along the y axis, as well as the possibility to move two pads, either 71 and 72, or 73 and 74, simultaneously in the same direction along the z axis. This allows alternatively, either to select track 3 as shown on figure 2a, or track 4 as shown on figure 2b, or track 5 as shown on figure 2c, or track 6 as shown on figure 2d.

Fig. 3a-3b-3c-3d show schematically respectively four different states of the internal translation mechanism, in another plane zx, of an example of a catheter robot according to an embodiment of the invention.

For example, the figure 3 illustrates the motion of the pads from a first situation where the track 6 is selected, on figure 3a, toward to a second situation where the track 4 is selected, on figure 3d. To change from track 6 selection on figure 3a to track 4 selection on figure 3d, the pair of pads 73 and 74 undergoes two intermediate states which are successively below former track 6 on figure 3b, and then after a move along axis x, below new track 4 on figure 3c.

The motorization (not represented for sake of simplicity) should be located under the pads 73 and 74, which means along the z axis, in the negative direction according to the axis shown on figure 3, because the other positions would interfere with the system. For instance, if motorization were to be located behind the pads 73 and 74, along the x axis, there would be an interference with the other tracks. For instance, if motorization were to be located above the pads 73 and 74, along the z axis, in the positive direction, their insertion and/or removal could not be done from the top of the system, which would be impractical. For instance, if motorization were to be located on the side of the pads 73 and 74, along the y axis, there would be an interference with the track and the flexible elongated medical element located within this track.

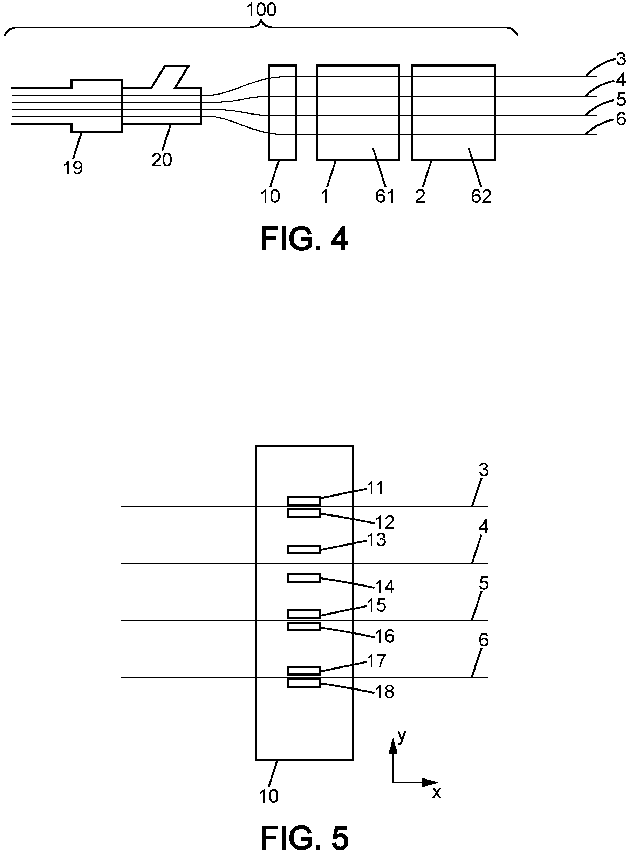

Fig. 4 shows schematically an example of a catheter robot according to an embodiment of the invention.

Coming back to figure 1, it can be seen that the non-robotized tracks are free, whereas it would be more secure if the flexible elongated medical elements located within these non-robotized tracks could be held motionless.

This can be obtained thanks to a motorized clamping device 10 placed in front of the first catheter translation module 1, the distal catheter translation module closest to the patient. The catheter robot then successively comprises the catheter guide 19, the Y valve 20, the clamping device 10, the distal catheter translation module 1, and the proximal catheter translation module 2.

Fig. 5 shows schematically an example of a clamping device of a catheter robot according to an embodiment of the invention.

The catheter robot comprises a clamping device 10. This clamping device 10 is longitudinally spaced apart from said first 1 and second 2 catheter translation modules.

This clamping device 10 comprises a group of several crossing tracks 3, 4, 5 and 6, within which several corresponding flexible elongated medical elements can translate through either distal catheter translation module 1 and/or proximal catheter translation module 2.

This group of crossing tracks 3, 4, 5 and 6, faces at least one of said groups of crossing tracks 3, 4, 5 and 6, of catheter translation modules 1 and 2, so that a given flexible elongated medical element which translates within a crossing track 3, 4, 5 and 6, of both groups of these catheter translation modules 1 and 2, also translates within a corresponding crossing track 3, 4, 5 and 6, of the group of this clamping device 10.

This clamping device 10 is synchronized with the catheter translation modules 1 and 2:
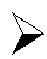 first, so as not to clamp in any crossing track 3, 4, 5 or 6, facing a corresponding crossing track 3, 4, 5 or 6, of the catheter translation modules 1 or 2 within which any of said sets 7 or 8 of two pairs of movable pads, 71 to 74 or 81 to 84, is directed by the switches 61 or 62,
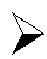 second, so as to clamp in one or more crossing tracks 3, 4, 5 and 6, not facing a crossing track 3, 4, 5 or 6, of the catheter translation modules 1 or 2 within which any of these sets 7 or 8 of two pairs of movable pads, 71 to 74 or 81 to 84, is directed by the switches 61 or 62.

Regarding the clamping device 10, several embodiments are possible. In a first embodiment, it is composed of 4 pairs of pads 11, 12, 13 and 14, with a motion along the y axis. On figure 5, the situation, which is illustrated, corresponds to flexible elongated medical elements located within tracks 3, 5 and 6 which are clamped, whereas flexible elongated medical element located within track 4 is unclamped.

The clamping device 10 comprises a cam mechanism disposed so as:
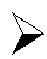 first, to require motor energy to change the status of anyone of said crossing tracks 3, 4, 5 or 6, from clamped to unclamped or from unclamped to clamped,
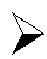 second, not to require motor energy to maintain the status of anyone of said crossing tracks, 3, 4, 5 or 6, either clamped or unclamped.

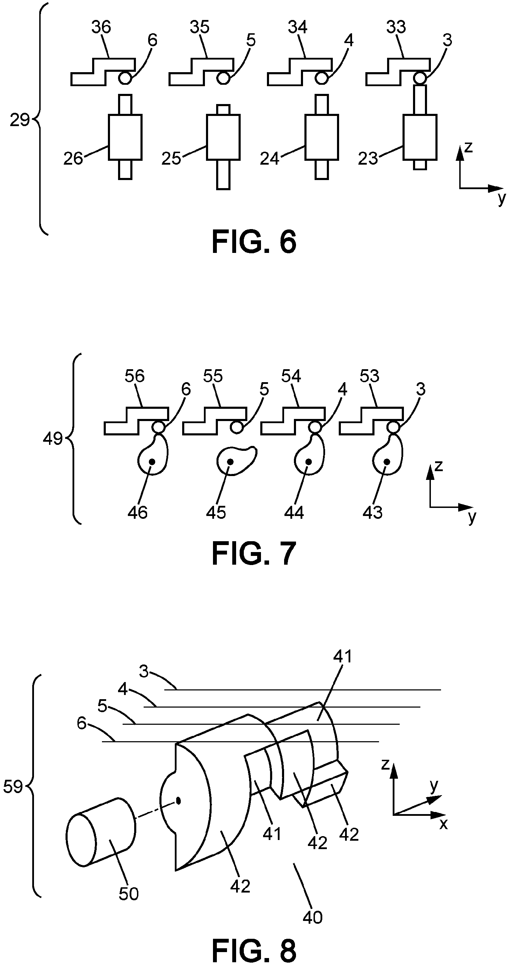

Fig. 6 shows schematically a first example of an internal clamping mechanism of a clamping device of a catheter robot according to an embodiment of the invention.

In the embodiment shown in figure 6, a cam mechanism 29 comprises:
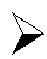 a finger composed of a linear motor is placed beneath each flexible elongated medical element to clamp, motor 23 for flexible elongated medical element located within track 3, motor 24 for flexible elongated medical element located within track 4, motor 25 for flexible elongated medical element located within track 5 and motor 26 for flexible elongated medical element located within track 6,
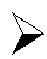 whereas a fixed counter-form, counter-form 33 for flexible elongated medical element located within track 3, counter-form 34 for flexible elongated medical element located within track 4, counter-form 35 for flexible elongated medical element located within track 5, and counter-form 36 for flexible elongated medical element located within track 6, serves as a fulcrum for clamping.

In figure 6, again, the flexible elongated medical element located within track 4 is unclamped, whereas the flexible elongated medical elements located within tracks 3, 5 and 6 are clamped.

Fig. 7 shows schematically a second example of an internal clamping mechanism of a clamping device of a catheter robot according to an embodiment of the invention.

In the embodiment shown in figure 7, a cam mechanism 49 comprises several distinct cams 43, 44, 45 and 46, with respective protrusions which may be disposed at different angular positions along their parallel respective rotation axis, so as to be able:
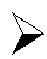 first, to clamp in one or more of the crossing tracks 3, 4, 5 and 6, at different rotation angle positions, in the plane zy,
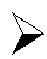 second, to clamp in different combinations of one or more of crossing tracks 3, 4, 5 and 6, respectively for different rotation angle ranges, in the plane zy, possibly to be able to clamp in each possible combination of any one or more of possible crossing tracks 3, 4, 5 and 6,
and which respective protrusions are able to clamp the flexible elongated medical elements respectively against corresponding counter-forms 53, 54, 55 and 56.

In the embodiment shown in figure 7, the linear motor of figure 6 has been replaced by a rotative motor that rotates a cam 43, 44, 45 or 46. Depending on the angle of the cam, 43, 44, 45 or 46, the device is clamped or unclamped. In figure 7, again the flexible elongated medical element located within track 4 is unclamped, whereas the flexible elongated medical elements located within tracks 3, 5 and 6 are clamped. As compared to the design of figure 6, this design does not require any energy to maintain the clamped or unclamped status. Indeed, the motors require energy only to rotate and change the status from clamped to unclamped or vice-versa.

Fig. 8 shows schematically a third example of an internal clamping mechanism of a clamping device of a catheter robot according to an embodiment of the invention.

In the embodiment shown in figure 8, a cam mechanism 59 comprises a single cam 40 with different diameters 41 and 42 disposed along its rotation axis y, so as:
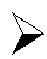 first, to clamp in only two crossing tracks, among tracks 3, 4, 5 and 6, at any rotation angle position,
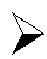 second, to clamp in different combinations of two crossing tracks, among tracks 3, 4, 5 and 6, respectively for different rotation angle ranges, each possible combination of two crossing tracks, among tracks 3, 4, 5 and 6, corresponding respectively to an associated rotation angle range.

In the embodiment shown in figure 8, there is only one long cam 40, rotating along the y axis. The shape of the cam 40 is configured in a way that, depending on the rotation angle, obtained thanks to the motor 50, any combination of 2 flexible elongated medical elements clamped / 2 flexible elongated medical elements unclamped can be obtained. The areas 42 have a larger cylinder diameter and correspond to the clamping positions, whereas the areas 41 with a smaller diameter correspond to the unclamped positions. For sake of simplicity, the corresponding counter-forms 53, 54, 55 and 56, also needed as on figure 7, are here not shown.

With the embodiment shown on figure 8, the order can be changed to obtain only one flexible elongated medical element (FEME in table 1) changed at a time when rotating the cam 40:

**TABLE 1**

| | Which FEME is clamped? | | | |
|---|---|---|---|---|
| Rotation angle (in degrees°) | FEME in track 3 | FEME in track 4 | FEME in track 5 | FEME in track 6 |
| 0-60 | YES | YES | NO | NO |
| 60-120 | YES | NO | YES | NO |
| 120-180 | YES | NO | NO | YES |
| 300-360 | NO | NO | YES | YES |
| 180-240 | NO | YES | YES | NO |
| 240-300 | NO | YES | NO | YES |

The invention has been described with reference to preferred embodiments. However, many variations are possible within the scope of the invention.

## Claims

1. Catheter robot comprising:
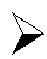 a base (100),
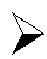 at least 2 catheter translation modules (1, 2) which are supported by said base (100),
∘ each catheter translation module (1, 2) comprising:
▪ only one set (7, 8) of movable parts (71 to 74, 81 to 84) so as to translate longitudinally at a time only one flexible elongated medical element,
▪ a group of several crossing tracks (3 to 6) within which several corresponding flexible elongated medical elements can translate through said catheter translation module (1, 2),
▪ a switch (61, 62) to direct said single set (7, 8) of movable parts (71 to 74, 81 to 84) in any of said crossing tracks (3 to 6) so as to then translate longitudinally said corresponding flexible elongated medical element,
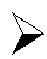 said first and second catheter translation modules (1, 2) being longitudinally spaced apart from each other so that:
∘ respective groups of crossing tracks (3 to 6) of both catheter translation modules (1, 2) are facing each other so that a given flexible elongated medical element which translates within a crossing track (3 to 6) of one group also translates within a crossing track (3 to 6) of the other group,
∘ both switches (61, 62) of said first and second catheter translation modules (1, 2) are synchronized together so as not to direct their respective sets (7, 8) of movable parts (71 to 74, 81 to 84) in crossing tracks (3 to 6) of said first and second groups which face each other.

2. Catheter robot according to claim 1, wherein it also comprises:
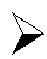 a clamping device (10) which is longitudinally spaced apart from said first and second catheter translation modules (1, 2) and which comprises:
∘ a group of several crossing tracks (3 to 6) within which several corresponding flexible elongated medical elements can translate through said catheter translation modules (1, 2),
∘ said group of crossing tracks (3 to 6) facing at least one of said groups of crossing tracks (3 to 6) of catheter translation modules (1, 2) so that a given flexible elongated medical element which translates within a crossing track (3 to 6) of both groups of said catheter translation modules (1, 2) also translates within a crossing track (3 to 6) of said group of said clamping device (10),
∘ said clamping device (10) being synchronized with said catheter translation modules (1, 2):
▪ so as not to clamp in any crossing track (3 to 6) facing a crossing track (3 to 6) of said catheter translation module (1, 2) within which any of said sets (7, 8) of two pairs of movable pads (71 to 74, 81 to 84) is directed by any of said switches (61, 62),
▪ so as to clamp in one or more or all crossing tracks (3 to 6) not facing a crossing track (3 to 6) of said catheter translation module (1, 2) within which any of said sets (7, 8) of two pairs of movable pads (71 to 74, 81 to 84) is directed by any of said switches (61, 62).

3. Catheter robot according to claim 2, wherein:
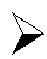 said clamping device (10) comprises a cam mechanism (29, 49, 59) disposed so as:
∘ to require motor energy to change the status of anyone of said crossing tracks (3 to 6) from clamped to unclamped or from unclamped to clamped,
∘ not to require motor energy to maintain the status of anyone of said crossing tracks (3 to 6), either clamped or unclamped.

4. Catheter robot according to claim 3, wherein:
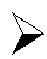 said cam mechanism (29, 49, 59) comprises a single cam (59) with different diameters (41, 42) disposed along its rotation axis, so as:
∘ to clamp in only two crossing tracks (3 to 6) at any rotation angle position,
∘ to clamp in different combinations of two crossing tracks (3 to 6) respectively for different rotation angle ranges, each possible combination of two crossing tracks (3 to 6) corresponding respectively to an associated rotation angle range.

5. Catheter robot according to any of preceding claims, wherein each catheter translation module (1, 2) is also a catheter rotation module so as to rotate around longitudinal axis said flexible elongated medical element.

6. Catheter robot comprising:
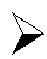 a base (100),
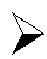 at least 2 catheter translation modules (1, 2) supported by said base (100),
∘ each catheter translation module (1, 2) comprising:
▪ only one set (7, 8) of two pairs of movable pads (71 to 74, 81 to 84):
• said pads (71 to 74, 81 to 84) of a same pair at least partly facing each other,
• alternatively, each of said two pairs clamping between its pads (71 to 74, 81 to 84) a flexible elongated medical element so as to then translate longitudinally said flexible elongated medical element,
▪ a group of several crossing tracks (3 to 6) within which several corresponding flexible elongated medical elements can translate through said catheter translation module (1, 2),
▪ a switch to direct said single set (7, 8) of two pairs of movable pads (71 to 74, 81 to 84) in any of said crossing tracks (3 to 6) so as to then translate longitudinally said corresponding flexible elongated medical element,
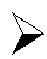 said first and second catheter translation modules (1, 2) being longitudinally spaced apart from each other so that:
∘ respective groups of crossing tracks (3 to 6) of both catheter translation modules (1, 2) are facing each other so that a given flexible elongated medical element which translates within a crossing track (3 to 6) of one group also translates within a crossing track (3 to 6) of the other group,
∘ both switches (61, 62) of said first and second catheter translation modules (1, 2) are synchronized together so as not to direct their respective sets (7, 8) of two pairs of movable pads (71 to 74, 81 to 84) in crossing tracks (3 to 6) of said first and second groups which face each other.

7. Catheter robot according to claim 6, wherein it also comprises:
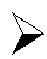 a clamping device (10) which is longitudinally spaced apart from said first and second catheter translation modules (1, 2) and which comprises:
∘ a group of several crossing tracks (3 to 6) within which several corresponding flexible elongated medical elements can translate through said catheter translation module (1, 2),
∘ said group of crossing tracks (3 to 6) facing at least one of said groups of crossing tracks (3 to 6) of catheter translation modules (1, 2) so that a given flexible elongated medical element which translates within a crossing track (3 to 6) of both groups of said catheter translation modules (1, 2) also translates within a crossing track (3 to 6) of said group of said clamping device (10),
∘ said clamping device (10) being synchronized with said catheter translation modules (1, 2):
▪ so as not to clamp in any crossing track (3 to 6) facing a crossing track (3 to 6) of said catheter translation module (1, 2) within which any of said sets of two pairs of movable pads (71 to 74, 81 to 84) is directed by any of said switches (61, 62),
▪ so as to clamp in one or more crossing tracks (3 to 6) not facing a crossing track (3 to 6) of said catheter translation module (1, 2) within which any of said sets (7,8) of two pairs of movable pads (71 to 74, 81 to 84) is directed by any of said switches (61, 62).

8. Catheter robot according to claim 7, wherein:
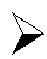 said clamping device (10) comprises a cam mechanism (29, 49, 59) disposed so as:
∘ to require motor energy to change the status of anyone of said crossing tracks (3 to 6) from clamped to unclamped or from unclamped to clamped,
∘ not to require motor energy to maintain the status of anyone of said crossing tracks (3 to 6), either clamped or unclamped.

9. Catheter robot according to claim 8, wherein:
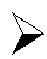 said cam mechanism (29, 49, 59) comprises a single cam (59) with different
diameters (41, 42) disposed along its rotation axis, so as:
∘ to clamp in only two crossing tracks (3 to 6) at any rotation angle position,
∘ to clamp in different combinations of two crossing tracks (3 to 6) respectively for different rotation angle ranges, each possible combination of two crossing tracks (3 to 6) corresponding respectively to an associated rotation angle range.

10. Catheter robot according to any of claims 6 to 9, wherein:
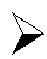 each catheter translation module (1, 2) is also a catheter rotation module:
∘ alternatively, each of said two pairs clamping between its facing pads (71 to 74, 81 to 84) a flexible elongated medical element so as to then rotate around longitudinal axis said flexible elongated medical element, by making said flexible elongated medical element rolling between its facing pads (71 to 74, 81 to 84).

11. Catheter robot according to any of preceding claims, wherein said group of several crossing tracks (3 to 6) comprises at least 4 crossing tracks (3 to 6), and preferably comprises only 4 crossing tracks (3 to 6).

12. Catheter robot according to claim 11, wherein:
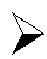 said crossing tracks (3 to 6) receive respectively:
∘ a first catheter, preferably a catheter balloon or a catheter stent,
∘ a first guide of said first catheter,
∘ a second catheter, preferably a catheter balloon or a catheter stent,
∘ a second guide of said second catheter.

13. Catheter robot according to any of preceding claims, wherein said first and second catheter translation modules (1, 2) are structurally identical to each other.

14. Catheter robot according to any of preceding claims, wherein each track (3 to 6) of one or more or all of the catheter translation modules (1, 2) is flexible elongated medical element agnostic so that said track can handle either a catheter or a guidewire.
